Europäisches Patentamt

European Patent Office

Office européen des brevets

(19)

(11) Publication number: **0 137 608**
**A2**

(12) **EUROPEAN PATENT APPLICATION**

(21) Application number: **84305198.8**

(22) Date of filing: **31.07.84**

(51) Int. Cl.⁴: **A 61 L 15/00**

(30) Priority: **11.08.83 US 522874**
**07.12.83 US 559156**

(43) Date of publication of application:
**17.04.85 Bulletin 85/16**

(84) Designated Contracting States:
**AT BE CH DE FR IT LI LU NL SE**

(71) Applicant: **THE PROCTER & GAMBLE COMPANY**
**301 East Sixth Street**
**Cincinnati Ohio 45201(US)**

(72) Inventor: **Rich, Thomas Floyd**
**11375 Kenshire Drive**
**Cincinnati Ohio 45240(US)**

(74) Representative: **Gibson, Tony Nicholas et al,**
**Procter & Gamble (NTC) Limited Whitley Road**
**Longbenton Newcastle upon Tyne NE12 9TS(GB)**

(54) Absorbent structures comprising vegetable absorbent material and disposable diapers incorporating such structures.

(57) Absorbent structures comprising from 1% to 99% of a vegetable-derived pectin-containing absorbent material and from 1% to 99% of a conventional absorbent material are disclosed.

EP 0 137 608 A2

Croydon Printing Company Ltd.

ABSORBENT STRUCTURES COMPRISING VEGETABLE
ABSORBENT MATERIAL~~AND DISPOSABLE DIAPERS~~
INCORPORATING ~~SUCH STRUCTURES~~

Thomas F. Rich

## TECHNICAL FIELD

This invention relates to novel absorbent structures comprising absorbent materials of vegetable origin.

Disposable absorbent products (e.g. disposable diapers, sanitary napkins, and the like) generally contain absorbent webs of wood pulp fibers. Depending on the climate, it takes a tree from about 20 years to about 40 years to mature to a harvestable state. Consequently, to meet the demand of wood pulp fibers to be used in absorbent materials, vast areas of land are necessary for the growing of trees. As another consequence, even though wood pulp fibers are probably the most economical material for disposable absorbent products available today, substantial expenditures are being made in the harvesting and growing of the trees. There is therefore a continuing need for alternative, inexpensive absorbent materials, preferably from renewable resources.

It has now been discovered that certain pectin-containing agricultural by-products can be converted to highly absorbent materials, suitable for use in disposable absorbent products, via a relatively simple and inexpensive process. Typical examples of agricultural by-products suitable as raw materials for the absorbent materials of the present invention include the residue material from citrus juice processors and from sugar beet refineries. These materials are therefore abundantly available at low cost.

The agricultural by-products are converted to absorbent materials by a process preferably involving deesterification of the pectin methyl esters in the material, removal of water soluble organic materials (e.g. carbohydrates) or both. Preferred vegetable derived materials are prepared by a process involving hydrolysis or partial hydrolysis of the pectin methyl esters present in waste materials; and subsequent washing and drying of the material. The absorbent properties of the materials may be further improved by subjecting them to a bleaching step. Such a bleaching step also improves the appearance of the product, thereby making it more acceptable for use in disposable absorbent products. The vegetable-derived absorbent materials typically have an absorbent capacity which is from 2 to 5 times that of conventional wood pulp fiber webs. These materials therefore offer an opportunity to reduce the bulk of absorbent products while maintaining their containment capacity.

It is therefore an object of this invention to provide absorbent structures which comprise, in addition to a conventional absorbent material, a novel absorbent material of vegetable origin. It is a further object of this invention to provide disposable absorbent products, like disposable diapers and sanitary napkins, comprising the absorbent structure of the present invention.

## Background of the Invention

The agricultural waste materials suitable as starting materials for the manufacture of the absorbent materials of the present invention are being produced in large quantities. The waste material from citrus juice processors, consisting of peels (i.e. albedo and flavedo) and rag, is generally processed to cattle feed by what has been termed the "lime de-watering process". This process comprises the steps of treating the waste with "lime" (calcium oxide, calcium hydroxide, or even calcium carbonate) to convert it from a slimy, unpressable condition to a watery, pressable condition; pressing the converted waste; and drying (see, for example, U.S. Patent 2,147,521, issued February 14, 1939 to Florida Citrus Exchange; U.S. Patent 2,215,944, issued September 24, 1943 to Vincente; U.S. Patent 2,362,014, issued November 7, 1944 to Citrus Processes, Inc.).

Relatively small quantities of citrus waste are used as a source of pectin, which can be used as a thickening agent in food products. Food thickening agents may also be prepared by comminuting citrus peel material, and lowering the degree of esterification of the pectic materials in the citrus peel by enzymatic or chemical treatment. This approach has been disclosed in U.S. Patent 3,982,003 issued September 21, 1976 to Mitchell et al., and in U.S. Patent 4,143,172, issued March 6,

10. An absorbent structure according to any one of claims 1-9 wherein the conventional absorbent material is selected from absorbent fibers, water-insoluble hydrogels, and mixtures thereof.

11. An absorbent structure according to claim 10 wherein the absorbent fiber is cellulose fiber, preferably wood pulp fiber.

12. An absorbent structure according to claim 11 comprising from 5% to 80% vegetable absorbent material, and from 20% to 95% wood pulp fiber.

13. An absorbent structure according to either one of claims 10 and 11 comprising from 50% to 95% vegetable absorbent material and from 5% to 50% of a water-insoluble hydrogel.

14. An absorbent structure according to either one of claims 10 and 11 comprising from 40% to 60% wood pulp fiber, from 20% to 40% vegetable absorbent material, and from 10% to 25% water-insoluble hydrogel.

15. An absorbent structure according to any one of claims 10-14 which comprises, as an additional component, from 0.5% to 5% of a long thermoplastic fiber.

16. An absorbent structure according to any one of claims 10 and 13-15 wherein the water-insoluble hydrogel is selected from hydrolized acrylonitrile grafted starch, acrylic acid grafted starch, polyacrylates, copolymers of isobutylene and maleic anhydride, and mixtures thereof.

17. An absorbent structure according to any one of claims 12-15 which is a web of conventional absorbent material, having dispersed therein the vegetable absorbent material in particulate form.

18. An absorbent structure according to any one of claims 12-15 which comprises a sheet of vegetable absorbent material and a conventional absorbent material fiber web.

19. An absorbent structure according to any one of claims 12-18 having a density of from 0.2 to 1 $g/cm^3$, preferably from 0.3 to 0.5 $g/cm^3$.

20. A disposable diaper, incorporating an absorbent structure in accordance with any one of claims 1-19.

21. A disposable diaper according to claim 20 wherein the absorbent structure is wrapped in envelope tissue.

by this analytical protocol, except "equivalent percent divalent metals" and "degree of esterification", which are equivalent percentages of the total amount of polygalacturonic acid. Data obtained by a different analytical method, however, may differ, which precludes a direct comparison with such data. In particular the pectin, cellulose and hemicellulose contents and the degree of esterification which are determined indirectly, are parameters which are sensitive to the analytical method used.

In the analytical protocol the water content of a sample of the material to be analyzed is determined by Karl Fischer titration. The amounts of calcium, magnesium, sodium and potassium are determined by dry ashing followed by flame atomic absorption analysis of these metals. A third sample of the material is subjected to water extraction followed by a 40-hour Soxhlet extraction with chloroform. The amount of lipids in the extract is determined gravimetrically (hereinafter referred to as "chloroform soluble lipids"). If the material to be analyzed does not contain components which are both water-soluble and chloroform-soluble, the water extraction prior to the chloroform extraction may be omitted. Thus, it has been found that a chloroform extract of citrus peel derived absorbent materials does not contain water-soluble components, but that a sugar beet derived absorbent material does contain water-soluble components in the chloroform extract. A citrus peel-derived material may therefore be extracted with chloroform without prior water extraction, and an accurate reading of the amount of chloroform soluble lipids is obtained; a sugar beet derived material, on the other hand, must be extracted with water prior to chloroform extraction in order to obtain an accurate determination of the amount of chloroform soluble lipids.

A fourth sample is extracted with chloroform, and is subsequently subjected to a 40 hour Soxhlet extraction with chloroform/methanol/water (20/4/1, v/v/v). (If the determination of chloroform soluble lipids was made without prior water extraction the residue of that test can be used for this chloroform/methanol/water extraction). The amount of extracted

material, which is determined gravimetrically, is the sum of the amounts of water-soluble metal salts and non-lipid organic materials. The metal cations are determined by flame ionization; the amount of water-soluble metal salts (hereinafter referred to as "water-soluble metal salts") is calculated therefrom using the molecular weight of the prevalent anion (which is known from the processing history of the sample; in case of chlorine bleaching, for example, the prevalent anion is chloride). The amount of non-lipid organic materials (hereinafter referred to as "non-lipid organic materials") is determined by subtracting the amount of water-soluble metal salts from the total amount of material extracted with the chloroform/methanol/water mixture.

The residue of the chloroform/methanol/water extraction contains cellulose, hemicellulose, lignin, pectin and pectates. The residue is split in four parts; one part is subjected to an acid hydrolysis of cellulose and hemicellulose, followed by GC analysis of the aldononitrileperacetate derivatives of the sugars. The second part of the residue is analyzed for protein; the protein content is calculated from the nitrogen value which is determined colorimetrically using the Nessler reagent. The third part of the residue is analyzed for lignin, using the gravimetrical method TAPPI T222-os-74. The fourth part of the residue is analyzed for calcium, magnesium, sodium and potassium by dry ashing followed by flame atomic absorption analysis of the metals. The amount of pectin is calculated from the total amount of bound metals (i.e., metals which cannot be removed by chloroform extraction or chloroform/methanol/water extraction) and the methoxy content. From the amounts of bound calcium and magnesium and the amount of pectin in the sample, the equivalent percent divalent metals is calculated as the equivalent percent of the polygalacturonic acid which is present as divalent metal salt. Likewise, the degree of esterification is calculated as the equivalent percent of the polygalacturonic acid which is present as the methyl ester, from the methoxy content and the total amount of pectin in the sample.

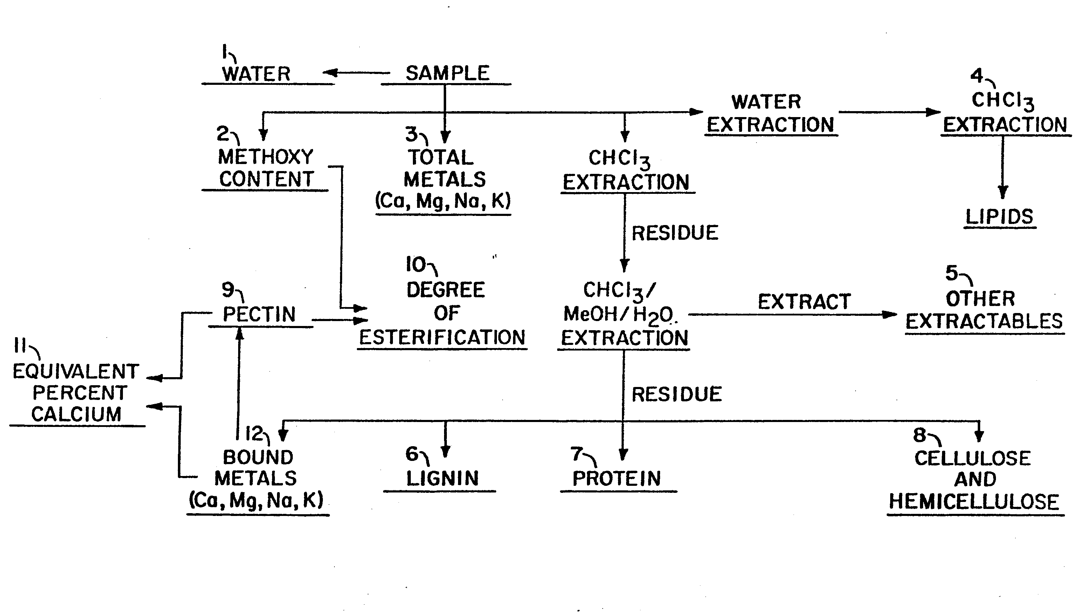

The analytical protocol is illustracted by the flow chart of Figure 1. The water content 1 is determined by Karl Fischer titration. The methoxy content 2 is determined by base hydrolysis followed by GC analysis of the liberated methanol. Total metals 3 are determined by dry ashing followed by flame atomic absorption analysis of the metals. Lipids 4 are determined by gravimetry after a 40-hour Soxhlet extraction with chloroform. The residue of the chloroform extraction is then subjected to a 40-hour Soxhlet extraction with chloroform/methanol/water (20/4/1, v/v/v), other extractables 5 are determined gravimetrically. Lignin 6 is determined by gravimetry using TAPPI method T222-os-74. Protein 7 is calculated from the nitrogen value which is determined colorimetrically using the Nessler reagent. Cellulose and hemicellulose 8 are determined by acid hydrolysis followed by GC analysis of the aldononitrileperacetate derivatives of the sugars. Bound metals 12 are determined by dry ashing followed by flame atomic absorption analysis of the metals. Pectin 9 is calculated from the sum of the methoxy content 2 and bound metals 12. From bound calcium 12 and pectin 9 is calculated the equivalent percent calcium 11. From methoxy content 2 and pectin 9 is calculated the degree of esterification 10 as equivalent percent of polygalacturonic acid which is present as the methyl ester.

"Pectin", as determined by this method is the material in the composition which is capable of forming methyl esters or binding metals, calculated as polygalacturonic acid. The underlying assumptions are that the pectin does not contain any free acid groups, that all of the pectin is anhydrogalacturonic acid, and that no insoluble alkaline earth metal salts, other than calcium salts, are present. These assumptions have been verified by independent methods to be correct within a reasonable margin of confidence.

The sum of cellulose and hemicellulose is taken to be the total of neutral sugars left in the sample after the extractions with chloroform and the chloroform/methanol/water mixture.

Cellulose may be determined separately as the total amount of glucose. The balance of neutral sugars is hemicellulose.

It has been discovered that the pectin in the composition plays an important part in determining the absorbent properties of the material. Although compositions differ among species and within species, more than 60% of the pectin in vegetable materials is generally present in the form of the methyl ester. In the case of orange peels, on the order of 20% is present as the calcium salt; the balance is generally in the protonated form, or an alkali metal salt, mostly potassium. Preferred vegetable-derived absorbent materials for use in the absorbent structures of the present invention are materials in which the pectin has a degree of esterification of less than 45%, more preferably less than 20%. In order to obtain a degree of esterification of less than 45%, the the vegetable starting material must be subjected to a deesterification step which may be carried out by alkaline treatment at a pH of from 8 to 13, or by an enzyme such as pectin-esterase. The enzyme is naturally present in citrus peel. Care should be taken that the amount of divalent metal pectates is not substantially increased. In particular, calcium pectates have been found to be detrimental to the absorption properties of the material. Moreover, the calcium pectates once formed cannot be readily converted to other pectic materials like alkali metal salts or pectic acids. In general, the total equivalent % of divalent metals must be less than 50%. The equivalent % of calcium is preferably less than 30%. In practical terms this means that calcium hydroxide or calcium carbonate cannot be used for alkaline deesterification. It is not necessary, however, to use distilled or deionized water: tap water has been found to not significantly reduce the absorbent properties of the materials, provided the water hardness does not exceed 7 grains/gallon (corresponding to 120 ppm $CaCO_3$) and provided that no excessive amounts of water are used. The term "soft water" as used herein therefore refers to water having a degree of hardness of less than 7 grains/gallon (less than 120 ppm $CaCO_3$).

- 9 -

In particular when citrus waste is used, the vegetable starting material may contain complex mixtures of lipids and lipid-like materials, and other non-polymeric organic materials. The absorbent properties of the resulting absorbent material may be greatly enhanced by removing these organic extractable materials. It has been discovered that the organic extractables generally belong to one of two classes: a first class of materials which are soluble in chloroform and which have been identified as mostly non-polar lipids (these materials are referred to herein as "chloroform soluble lipids"); and a second class of materials which are not soluble in chloroform alone, but which are soluble in a mixture of chloroform, methanol and water (chloroform:methanol: water = 20:4:1 (v/v/v)). This second class of materials is comprised of non-lipid organic materials and of water-soluble metal salts.

For use in the absorbent structures of the present invention, the vegetable-derived absorbent materials may not contain more than 4% chloroform soluble lipids, preferably less than 1%. It is further critical that the vegetable-derived absorbent material not comprise more than 35% of non-lipid organic materials extractable in a mixture of chloroform, methanol and water (chloroform:methanol:water = 20:4:1 (v/v/v). Preferably, the vegetable-derived absorbent material contains less than 10% of such non-lipid organic materials. It is also preferred that the vegetable-derived absorbent materials comprise less than 6% water-soluble metal salts.

Hence, the present invention relates to absorbent structures comprising (1) from 1% to 99% of a vegetable-derived absorbent material which comprises by weight of the vegetable-derived absorbent material (a) from 15% to 60% pectin, less than 50% of which is in the form of a divalent metal salt; (b) from 15% to 80% of a material selected from cellulose, hemicellulose, lignin, and mixtures thereof; (c) from 0% to 4% chloroform soluble lipids; (d) from 0% to 35% non-lipid organic materials extractable in a mixture of chloroform, methanol and water, said mixture having a volume ratio chloroform:methanol:water of 20:4:1; and (2) from 1% to 99% of a conventional absorbent material.

Although the degree of esterification of the pectin does not appear to have a major effect on the ultimate absorption capacity of the absorbent material, low degrees of esterification are conducive to good wicking properties of the material. High wicking rates are important for a fast uptake of liquid. For use in absorbent products like disposable diapers, sanitary napkins and the like, good wicking properties of the absorbent material are highly desirable. The absorbent materials therefore preferably contain pectin which has a degree of esterification of less than 45%, more preferably less than 20%.

The second major component of the vegetable-derived absorbent material, next to pectin, is generally a mixture of cellulose, hemicellulose and lignin. The actual composition of this mixture is to a large extent determined by the choice of the raw material, and to a lesser extent by the process. For example, hemicellulose is likely to be partially removed during processing, which increases the relative amounts of cellulose and lignin in the mixture. Depending on the raw material source, the amount of lignin may be very small. For example, citrus waste has a much lower lignin level than beet pulp; yet very good absorbent materials can be prepared from either starting material.

It has been discovered that divalent metal pectates, in particular calcium pectates, are far inferior to alkali metal pectates (e.g. sodium pectates) with regard to absorbent properties. This is probably due to the fact that divalent metal salts of pectin are "cross-linked", whereby the divalent metal ion serves as the link between two adjacent pectin molecules. This cross-linking is believed to prevent swelling of the pectin and to thereby reduce its absorbent capacity. Some of the pectin is naturally present as the calcium salt. Care must be taken not to increase the amount of calcium pectate any further. Therefore, during processing the materials should not be exposed to excessive amounts of calcium. If calcium is present in the form of an insoluble mineral salt which is not capable of interacting with the esterified pectin, the presence of calcium is probably not harmful at all.

One specific embodiment of this invention is an absorbent structure comprising: (1) from . 1% to 99% of a citrus peel-derived absorbent material which comprises, by weight of the citrus peel-derived absorbent material (a) from 30% to 60% pectin, said pectin having a degree of esterification of less than 20%, and less than 30% of the pectin being in the form of a calcium salt; (b) from 30% to 60% of a mixture of cellulose and hemicellulose; (c) from 0% to . 1% chloroform soluble lipids; (d) from 0% to 10% non-lipid organic materials extractable in a mixture of chloroform, methanol and water, said mixture having a volume ratio chloroform:methanol:water of 20:4:1; (e) from 0% to 6% water-soluble metal salts; and (2) from 1% to 99% of a conventional absorbent material.

A second specific embodiment of this invention is an absorbent structure comprising: (1) from 1% to 99% of a sugar beet-derived absorbent material which comprises, by weight of the sugar beet-derived material (a) from 15% to about 35% pectin, said pectin having a degree of esterification of less than 45% (preferably less than 20%), and less than 30% of the pectin being in the form of a calcium salt; (b) from 20% to 80% of a mixture of cellulose and hemicellulose; (c) from 0% to 1% chloroform soluble lipids; (d) from 0% to 10% non-lipid organic materials extractable in a mixture of chloroform, methanol and water, said mixture having a volume ratio chloroform:methanol:water of 20:4:1; (e) from 0% to 6% water-soluble metal salts; and (2) from . 1% to 99% of a conventional absorbent material.

### Process for Preparing the Vegetable-Derived Absorbent Material

The process necessary for preparing the vegetable-derived absorbent material from the pectin-containing starting material depends to a large extent on the starting material being used.

The process is aimed at removing excessive amounts of extractable materials, and preferably at reducing the degree of esterification of the pectin in the material as well.

For example, vegetable-derived absorbent materials may be obtained from citrus peels using the process described in U.S. Patent 3,982,003, issued September 21, 1976 to Mitchell et al..

The process described in this reference results in vegetable-derived absorbent material of acceptable quality, in particular when the flavedo is removed from the orange peels prior to processing, as described in Example 7 therein. However, the process described in Mitchell does not comprise a bleaching or a washing step. The products obtained by the Mitchell process therefore tend to contain relatively high levels of chloroform soluble lipids, and to be relatively dark in color.

Acceptable vegetable-derived absorbent materials are also obtained from citrus peels or sugar beet pulp using the process described in U.S. Patent 4,379,782, issued April 12, 1983 to Staub et al..

The process described in this reference results in absorbent material of acceptable quality, especially when sugar beet pulp is used as the starting material. However, the process described in Staub does not comprise a deesterification step. As a consequence, the materials possess relatively poor wicking properties.

Preferred is a process which comprises the steps of (a) comminuting a pectin-containing vegetable material to a particle size of from 0.05 mm to 3 mm; (b) de-esterifying the pectin to a degree of esterification of less than 45%; (c) washing the vegetable material in soft water; and (d) drying the vegetable material to a moisture content of less than 15%.

The manner in which the deesterification is carried out is not critical and may, for example, be enzymatic or chemical.

Chemical deesterification of pectin may be carried out at acid or alkaline pH. Acid deesterification is not preferred as it is slow and leads to both divalent ion extraction and hemicellulose degradation. Alkaline deesterification is preferred but care must be exercised. Pectin methyl ester can degrade via a beta-elimination mechanism, so pH and temperature should be carefully controlled. Enzymatic deesterification may be carried out with the enzyme pectinesterase; this is particularly convenient when citrus peels are used as starting material because the enzyme pectinesterase is naturally present in citrus peels. Alternatively, deesterification may be carried out by soaking the vegetable material particles in a solution of an alkali metal hydroxide. The reaction rate increases with the concentration of hydroxyl ions, therefore the higher the pH, the faster the reaction will be. The pH should therefore be above about 8, preferably above 9. Excessively high pH values tend to result in removal of hemicellulose and other desirable materials. Therefore, the pH should not exceed 13, and preferably be below 12. A degree of esterification of less than 45% is generally achieved after 2 minutes at pH 9.5 or greater. As beta-elimination is very temperature dependent, a temperature of 25°C or lower is preferred. Prolonged contacting of the vegetable material with the alkali hydroxide solution results in a progressively lower degree of esterification of the pectin. It is generally not necessary to continue this deesterification step for more than 2 hours, since little additional benefit is obtained by doing so.

The washing of the material is very important, since it is necessary to remove the alkaline material and excess soluble materials. Washing may be done with water or with an organic solvent. The latter has the advantage that chloroform-soluble lipids which may be present in the material are generally to some extent removed by an organic solvent like acetone, but not by a water wash. The washing step may be conveniently carried out as follows. Excess liquid is drained off from the reaction mixture of the deesterification step. Then, enough of the washing liquid (i.e. water or an organic solvent) is added in order to obtain a slurry with a solids content of 2%. The slurry is equilibrated for 5 to 15 minutes, and then the washing liquid is drained off. This washing step may be repeated. The number of washing steps is determined by the amount of contaminants in the starting material and the desired composition of the finished absorbent material. Typically, 2 or 3 washing steps will be necessary.

After excess washing liquid has been drained off after the last washing step, the liquid content of the remaining material is on the order of 90%. This must be reduced to less than 20% in order to obtain an absorbent material with optimum absorption properties. If an organic solvent is used in the washing step (e.g. acetone, isopropyl alcohol, or methanol), this solvent may be simply removed by evaporating it. The material may also be dried by solvent displacement: after the last washing step with water, the material is slurried up with an organic solvent like acetone or methanol, the solvent is then drained off and the excess is evaporated.

For economic reasons it may not be feasible to use any organic solvents in the process. In that case, water will have to be removed using a conventional drying technique. This may be either freeze drying, vacuum drying, or thermal drying. Of these three, freeze drying is the most attractive because it does not cause collapse of the fine capillaries which are naturally present in the material. Unfortunately, freeze drying is also the most expensive drying method of these three.

Thermal drying is economically the most attactive method of drying available. If the 90% moisture product from the washing step is dried in an oven, a board-like material is obtained which is not very absorbent. It has been discovered, however, that the material may be dried by spraying it into a counter current of superheated steam or heated air. The inlet temperature and the flow rate should be controlled as to result in an outlet temperature in the range of from 60°C to 75°C. This results in a product which has a moisture content of less than 15%. Although thermally dried materials possess good absorption capacities, the absorption kinetics of such materials tend to be slow. The rate of absorption can be vastly improved by adding a surfactant to the slurry during the last washing step. An amount of surfactant of from 1% to 3% by weight of the amount of the slurry is generally sufficient. The type of surfactant is not critical. Examples of suitable surfactants are nonionic surfactants, e.g. ethoxylated fatty alcohols.

It is highly desirable to include in the process a bleaching step. Preferably, the bleaching is carried out with an oxidative bleaching agent. Examples of suitable bleaching agents are hydrogen peroxide, perborate, hypochlorite, chlorine dioxide and chlorine. Sodium hypochlorite is a preferred bleaching agent. For optimum properties of the absorbent material, and optimum safety of absorbent products made therewith, excess bleach and electrolytes introduced during the bleaching step will have to be removed. Bleaching is therefore best carried out prior to the washing steps. Since color compounds may be formed during alkaline deesterification, the bleaching step is best carried out subsequent to the deesterification step if alkaline deesterification is used (as opposed to enzymatic deesterification). In order to improve the effective use of the bleaching agent, it is desirable to include a washing step subsequent to the deesterification step and prior to the bleaching step, especially when citrus peels are the starting material. Hence, in case alkaline deesterification and bleaching are used, the process sequence is as follows: (a) communiting the pectin-containing vegetable material to a particle size of from 0.05 mm to 3 mm; (b) soaking the pectin-containing vegetable material particles obtained in step (a) in a solution of an alkali metal hydroxide in water at a pH of from 9 to 12 and at a temperature of from 15°C to 50°C, for a period of from 2 minutes to 120 minutes; (c) washing the product of step (b) in soft water; (d) bleaching the product of step (c) for from 5 minutes to 60 minutes; (e) washing the product of step (d) in soft water; and (f) drying the product of step (e).

The effect of bleaching is two-fold. It removes color materials, thereby vastly improving the appearance of the absorbent material obtained by this process, and making it more

suitable for use in consumer products like disposable diapers, sanitary napkins and the like. Bleaching further tends to decompose chloroform-soluble lipids into water-soluble fragments. Consequently, the bleaching step significantly reduces the level of chloroform-soluble materials in the finished product.

The removal of chloroform-soluble lipids is particularly important when citrus peels are used as the starting material. Citrus peels contain high levels of such chloroform-soluble lipids, and if water (as opposed to organic solvents) is used in the washing steps of the process, bleaching is instrumental in reducing the level of chloroform-soluble lipids to the desired level.

Although whole citrus peels may be used as the starting material, the flavedo part greatly increases the load of chloroform-soluble lipids and colored materials. It is therefore desirable to remove the flavedo part of the peel. The flavedo may be shaved off mechanically by machinery which is commercially available and designed for this purpose. Such equipment typically leaves about 30% of the flavedo on the albedo. It has been discovered that when these machine shaved peels are subjected to the process described hereinabove (including the bleaching step) an absorbent material is obtained which is negligibly less absorbent than the material obtained from an (handmade) all albedo starting material. Whole citrus peels may also be used as starting material. Highly acceptable absorbent materials may be made therefrom, albeit at the expense of a higher usage of bleaching chemicals.

Sugar beets, by their nature, contain only low levels of chloroform-soluble lipids. When processing sugar beet waste, one may therefore forego the bleaching step if absorbent properties are the only concern. However, during alkaline deesterification, beet pulp develops a persistent green color and a bleaching step may be highly desirable or even necessary from an aesthetics viewpoint.

The processing of other pectin-containing raw materials will have become apparent from the foregoing. The bleaching step may be foregone if the starting material has a low chloroform-soluble lipid content, and contains little colored materials or if the aesthetics of the absorbent material are relatively unimportant (e.g. when intended for industrial use). The choice of the method of deesterification (enzymatic or alkaline treatment) is largely determined by economic determinations: enzymatic deesterification is relatively slow; alkaline treatment is faster and lends itself better to a continuous operation of the process. The choice of the washing liquid (water or an organic solvent) is likewise determined by economic considerations with which a person skilled in chemical engineering may be deemed well familiar.

By "conventional absorbent material" herein is meant any absorbent material which is being used, or has been proposed for use, in absorbent products like disposable diapers, sanitary napkins, disposable towels, facial tissues, toilet tissues, incontinent pads, and the like. Examples therefore include absorbent fibers and water-insoluble hydrogels. Examples of absorbent fibers include vegetable fibers like cotton fibers, wood pulp fibers (e.g., Kraft pulp fibers, chemo-thermo mechanical pulp fibers), and fibers of abaca, sisal, henequen, cantala, istle, mauritirus, phornium, sansevieria, caroa, piassava, broomroot, flax, hemp, ramie, jute, kenaf, roselle, urena, coir and kapok. It should be noted that the above fibers, although probably encompassed by the dictionary meaning of "vegetable-derived absorbent materials", as defined herein do not fall under that term, but rather are encompassed by the term "conventional absorbent material". Examples of absorbent fibers further include man-made fibers like rayon, cellulose acetate, cellulose triacetate, alginate fibers, protein fibers, polyamide, nylon-6,6, nylon-6, aromatic polyamides, polyester, acrylic fibers, polyethene and polypropene fibers. Many of the man-made fibers are hydrophobic, but can be hydrophilized using art-disclosed techniques. Hydrophobic fibers may be hydrophilized by surfactant treatment, as disclosed in U.S. Patent 3,916,447, issued November 4, 1975, to Thompson; and in U.S. Patent 4,100,324, issued July 11, 1978 to Anderson et al..

Thermoplastic fibers may further be hydrophilized by coating with a hydrophilic material, e.g. silica, or by surface-grafting the fibers with hydrophilic groups.

By "hydrogel" as used herein is meant an inorganic or organic compound capable of absorbing aqueous fluids and retaining them under moderate pressures. For good results, the hydrogels must be water insoluble. Examples are inorganic materials such as silica gels and organic compounds such as cross-linked polymers. Cross-linking may be by covalent, ionic, vander Waals, or hydrogen bonding. Examples of polymers include polyacrylamides, polyvinyl alcohol, ethylene maleic anhydride copolymers, polyvinyl ethers, hydroxypropyl cellulose, carboxymethyl cellulose, polyvinyl morpholinone, polymers and copolymers of vinyl sulfonic acid, polyacrylates, polyacrylamides, polyvinyl pyridine and the like. Other suitable hydrogels are those disclosed in U.S. Patent 3,901,236, issued to Assarsson et al., August 26, 1975.

Particularly preferred polymers for use herein are hydrolyzed acrylonitrile grafted starch, acrylic acid grafted starch, polyacrylates, and isobutylene maleic anhydride copolymers, or mixtures thereof.

Processes for preparing hydrogels are disclosed in U.S. Patent 4,076,663, issued February 28, 1978 to Fusayoshi Masuda et al.; in U.S. Patent 4,286,082, issued August 25, 1981 to Tsuno Tsubakimoto et al.; and further in U.S. Patents 3,734,876, 3,661,815, 3,670,731, 3,664,343, 3,783,871, and Belgian Patent 785,858;

One specific embodiment of the present invention is an absorbent structure comprising a vegetable absorbent material and a conventional absorbent fiber. Preferred for use herein are cellulose fibers, in particular wood pulp fibers. In a preferred embodiment the absorbent structure comprises from 5% to 80% vegetable absorbent material, and from 20% to 95% wood pulp fiber.

The vegetable absorbent material and the conventional absorbent fiber can be combined in a variety of ways to form the absorbent structures of the present invention. For example, vegetable absorbent material can be mixed with conventional absorbent fibers, and the mixture formed into a web; or particulate vegetable absorbent material can be introduced into a web of conventional absorbent fibers in a certain pattern, so as to create areas of increased absorbent capacity within the absorbent structure; or the vegetable absorbent material can be pressed into a sheet which is then placed against a web, or sandwiched inbetween webs of the conventional absorbent fibers. Other executions will be apparent to those skilled in the art.

The vegetable absorbent material as obtained from the process described hereinabove is generally in particulate or fibrous form. The material can be put into sheet form by applying pressure to wet-laid material, and subsequent drying.

Another specific embodiment of the present invention is an absorbent structure comprising a vegetable-derived absorbent material, conventional absorbent fibers like wood pulp fibers, and a water-insoluble hydrogel, specifically polyacrylate grafted starch or slightly crosslinked polyacrylate material. Preferred are absorbent structures comprising from 40% to 60% wood pulp fiber, from 20% to 40% vegetable absorbent material and from 10% to 25% water-insoluble hydrogel.

The absorbent structures may conveniently be made by using conventional equipment designed for air laying of hydrophilic fibrous webs. In such equipment, webs are typically formed by taking up hydrophilic fibers in an air flow and depositing the fibers on a wire mesh screen. By metering the desired quantities of vegetable absorbent material particles or fiber particles into the air flow at a point just upstream of the wire mesh screen, the desired mixture of hydrophilic fibers and hydrogel particles can be made. The web formed on the screen is then passed through calender rolls which are set to a nip pressure resulting in the desired density of the absorbent structure . It will be clear that this embodiment of the process requires only minor modifications of conventional equipment for the manufacture of absorbent structures, i.e. installing a metering device for the addition of the vegetable absorbent material particles or fibers. In certain instances it may be necessary to replace the standard wire mesh screen on the equipment with one of a finer mesh size. This need will arise when relatively small hydrogel particles are used, and/or when the mesh size of the standard screen is relatively coarse.

Optionally, the structures may be compressed to a higher density than that of conventional air-laid wood pulp fiber webs (i.e., a density higher than : 0.1 g/cm³) by increasing the nip pressure on the calender rolls. The densified absorbent structures have good absorbent properties, in spite of the reduced void volume of such structures. This is due to the wet resiliency exhibited by the material. It regains virtually all of its original volume if wetted in a densified state and therefore exhibits its high absorbency in both an uncompressed and compressed state (unlike wood pulp fiber webs which become distinctly less absorbent upon compression). The densified structures therefore have properties (low bulk, high absorbency) which are highly desirable for absorbent products like disposable diapers, incontinent pads and sanitary napkins. The densified structures have a density of from 0.2 g/cm³ to 1 g/cm³, preferably from 0.3 to 0.5 g/cm³.

Alternatively, an absorbent structure may be formed by placing a web of absorbent fibers against a sheet of vegetable absorbent material. Optionally, the sheet and/or the web may be wrapped in envelope tissue, to increase the lateral strength of the structure.

Alternatively, an absorbent structure can be formed by mixing absorbent fibers, e.g., wood pulp fibers, and particles of the vegetable absorbent material in an aqueous slurry; admixing a surfactant; and foaming with air. The foamed slurry is then conveyed onto a wire screen and dewatered, preferably by applying vacuum to the underside of the wire screen. The foamed mat thus obtained is subsequently dried in air. A more detailed description of the foaming process is disclosed in U.S. Patent 3,871,952, issued March 18, 1975 to Robertson.

The absorbent structures of the present invention encompass structures comprising the vegetable absorbent material and a water-insoluble hydrogel. Examples include structures comprising a mixture of hydrogel particles or fibers and particles or fibers of the vegetable absorbent material; and laminates comprising one or more sheets of hydrogel material and one or more sheets of vegetable absorbent material, e.g. a laminate of one sheet of water-insoluble hydrogel placed against a sheet of vegetable absorbent material, or a "sandwich" type laminate, comprising a sheet of water-insoluble hydrogel material placed in between two sheets of vegetable absorbent material. Many variations are possible, as will be apparent to those skilled in the art.

A preferred embodiment is an absorbent structure comprising a mixture of from 50% to 95% vegetable absorbent material and from 5% to 50% of a water-insoluble hydrogel. Both the hydrogel and the vegetable absorbent material are preferably in particulate or fibrous form.

The mixture can be densified to a density of 1 g/cm³ without significant loss of absorption capacity. The densified structures have better wet strength and dry strength than non-densified structures. Moreover, the densified structures can be used for making low bulk absorbent products (like diapers and sanitary napkins) which are highly desirable from a consumer standpoint. Densified structures, having a density of from 0.2 to 1 g/cm³, are therefore preferred. More preferred are structures having a density of from 0.3 to 0.5 g/cm³.

Excellent absorbent structures are also obtained upon mixing of hydrophilic fibers, particles or fibers of the vegetable absorbent material, and particles or fibers of a water-insoluble hydrogel. The three components can be mixed in any ratio. Preferred are structures comprising from 30% to 80% hydrophilic fibers; from 10% to 50% vegetable absorbent material; and from 5% to 50% water-insoluble hydrogel. More preferred are structures comprising from 40% to 70% hydrophilic fibers; from 20% to 40% vegetable absorbent material; and from 5% to 25% water-insoluble hydrogel.

The performance of these three-component structures is improved by densifying the structures to a density of from 0.2 to 1 g/cm³, preferably from 0.3 to 0.5 g/cm³.

To improve the strength of the absorbent structures of the present invention the structures can be mixed with a small amount (typically from 0.5% to 5%) of a long thermoplastic fiber. As used herein, long fiber means a fiber having a length of more than 1 inch (· 2.5 cm). Suitable thermoplastic materials are inexpensive polymers like polyethylene, polypropylene and polyester. Polyester fibers are preferred because they are more hydrophilic than polyolefin fibers. The use of thermoplastic fibers in absorbent structures for the purpose of improving the strength of such structures is described in more detail in U.S. Patent 4,307,721, issued December 29, 1981 to Tsuchiya et al.,; U.S. Patent 4,219,024, issued August 26, 1980 to Patience et al.; and in U.S. Patent 4,100,329, issued July 11, 1978 to Anderson et al.,

Because of their particular properties, the absorbent structures of this invention are extremely suitable for use in disposable absorbent products. By "absorbent product" herein is meant a consumer product which is capable of absorbing significant quantities of water and other fluids, like body fluids. Examples of absorbent products include disposable diapers, sanitary napkins, incontinent pads, paper towels, facial tissues, and the like. The absorbent structures of the present invention are particularly suitable for use in products like diapers, incontinent pads, and

sanitary napkins. It is possible to design absorbent products which are thin and yet have more than sufficient absorbent capacity to avoid the embarrassment of failure. Flexibility of the structure ensures comfort for the wearer and a good fit of the absorbent product.

Disposable diapers comprising the absorbent structures of the present invention may be made by using conventional diaper making techniques, but replacing the wood pulp fiber web core which is typically used in conventional diapers with an absorbent structure of the present invention. Thus, a disposable diaper may be comprised of (from top to bottom) a top sheet (a non-woven, hydrophobic tissue, e.g. needle punched polyester), the absorbent structure, and a waterproof, pliable back sheet (e.g. hard polyethylene, having an embossed caliper of approximately 2.3 mils.). Optionally, the absorbent structure may be wrapped in envelope tissue (wet strength tissue paper). Disposable diapers of this type are disclosed in more detail in U.S. Patent 3,952,745, issued April 27, 1976 to Duncan; and in U.S. Patent 3,860,003, issued January 14, 1975 to Buell.

·n                                              The diaper may further comprise a second absorbent core, like a wood pulp fiber web, or a sheet of water-insoluble hydrogel.

Because the absorbent structures of the present invention are highly absorbent, and yet thin and flexible, they are extremely suitable for use in sanitary napkins. As is the case with disposable diapers, sanitary napkins utilizing the present absorbent structures may be derived from conventional sanitary napkins by simply replacing the absorbent core thereof (typically a web of wood pulp fibers) with an absorbent structure of the present invention. Such replacement may be on a weight-by-weight basis, which results in a reduction in volume and a gain in capacity; or the replacement may be on a less than equal weight basis, thereby sacrificing part of the gain in absorbent capacity in favor of an even greater reduction in bulk.

An example of a sanitary napkin comprises a pad of the absorbent structure of the present invention; a hydrophobic

- 24 -

topsheet; and a fluid impervious bottom sheet. The topsheet and the backsheet are placed at opposite sides of the absorbent structure. Optionally, the absorbent structure is wrapped in envelope tissue. Suitable materials for top sheets, bottom sheet and envelope tissue are well known in the art. A more detailed description of sanitary napkins and suitable materials for use therein is found in U.S. Patent 3,871,378, issued March 18, 1975 to Duncan et al.,

### Example One

Florida Valencia oranges juiced with an AMC Extractor were hand shaved to remove the flavedo and rag. The albedo was ground in an Urschel Grinder. Twenty-three pounds (about 10 kg) of the ground raw material, at 11.96% solids, was slurried in 75 pounds of water (about 34 kg), making a 2.8% solids slurry. The slurry was titrated to pH 9.5 and maintained at this pH by caustic addition for 30 minutes. The titration took 1.25 liters of 1N sodium hydroxide. The material was dewatered in a basket centrifuge. It was washed with 75 pounds (about 34 kg) of water while spinning in the centrifuge, and dewatered again. The 14.7 pounds (6.7 kg) of filter cake at 10.71% solids that resulted from this was slurried in 43 pounds (19.5 kg) of water, making a 2.8% solids slurry. 3.8 liters of 5.25% sodium hypochlorite solution were added to the slurry and mixed for 15 minutes. The bleached material was dewatered in the centrifuge. It was washed again with 75 pounds (about 34 kg) of water while spinning and then dewatered. This resulted in 12.2 pounds of material at 10.3% solids for a yield of 45.7%.

The material was freeze-dried to a moisture content of about 10%. The chemical composition was determined by the analytical method described hereinabove.

A. GROSS CHEMICAL COMPOSITION

| Component | Composition (% of dry weight) |
|---|---|
| Pectin | 44.0 |
| Other polymers | 52.7 |
| Chloroform soluble lipids | 0.70 |

| | |
|---|---|
| Non-lipid organics | 2.49 |
| Water-soluble metal salts | 0.13 |

B. DISTRIBUTION OF PECTIN COMPONENTS

| Component | Equivalent % polygalacturonic acid |
|---|---|
| Methyl ester | 14.4 |
| Divalent metal salts | 29.8 |
| Monovalent metal salts | 55.8 |

The citrus peel-derived absorbent material is mixed with wood pulp fibers in citrus peel material:wood pulp fiber ratios of 1:19, 1:9, 1:4, 1:3 and 1:1, respectively. The mixtures are formed into webs in a deckel box. The resulting absorbent structures possess excellent absorbent properties.

### Example Two

Four hundred thirty-four grams of finely ground sugar beet cattlefeed at 16.38% solids was slurried in 3 liters of water, making a 2.1% solids slurry. The slurry was titrated to pH 9.5 and maintained by caustic addition for 30 minutes. The titration took 12 ml of 1N sodium hydroxide. The material was dewatered in a basket centrifuge. It was washed with 50 pounds (22.7 kg) of water while spinning in the centrifuge, and then dewatered. The 462 grams of filter cake at 12.03% solids that resulted from this was slurried in 3 liters of water, making a 1.6% solids slurry. Five hundred mls of 5.25% sodium hypochlorite was added to this and it was mixed for 17 minutes. The bleached material was dewatered in the centrifuge. It was washed again with 50 pounds (22.7 kg) of water while spinning, and then dewatered. This resulted in 496 grams of material at 11.2% solids for a yield of 78%.

The material was dried by freeze-drying. Its chemical composition was determined by the method described hereinabove.

| Component | Composition (% of dry weight) |
|---|---|
| Pectin | 15.5 |
| Other polymers | 76.1 |
| Chloroform soluble lipids | 0.40 |
| Non-lipid organics | 6.79 |

Water soluble metal salts                                    1.60

B.  DISTRIBUTION OF PECTIN COMPONENTS

|  | Equivalent % polygalacturonic acid |
| --- | --- |
| Component | |
| Methyl ester | 22.9 |
| Divalent metal salts | 25.4 |
| Monovalent metal salts | 51.7 |

The sugar beet absorbent material is mixed with northern softwood Kraft pulp fibers and northern softwood chemo-thermo-mechanical pulp (CTMP) fibers in the following ratios:

|  | A | B | C | D |
| --- | --- | --- | --- | --- |
| Sugar Beet Absorbent Material (wt %) | 5 | 5 | 25 | 30 |
| Kraft pulp fibers (wt %) | 90 | 80 | 65 | 40 |
| CTMP fibers (wt %) | 5 | 15 | 10 | 30 |

The mixtures are formed into webs, using conventional air-laying equipment.  The resulting structures possess excellent absorbent properties.

### Example Three

Citrus peel absorbent material prepared as described in Example One was dry mixed with southern soft wood Kraft pulp fibers, in a ratio of 2:3.  Webs having dimensions of 15 x 25 cm, were prepared in batch type air laying equipment.  The webs were compressed to a dry density of 0.3 g/cm³, using a flat hydraulic press, corresponding to a thickness of 2.6 mm.  The absorbent structures had a weight of 30 g, and a final basis weight of 800 g/m².

Disposable diapers utilizing the above absorbent structure were prepared as follows.

The absorbent structures were enveloped in wet strength tissue paper having a basis weight of        12 pounds per 3,000 square feet (about 20 g/m²), a dry tensile strength of        700 g/inch (      275 g/cm) in the machine direction and        300 g/inch (     120 g/cm) in the cross machine direction.

The enveloped pad was glued onto a 7 in. x 11 in. (      18 cm x 28 cm) backsheet of embossed polyethylene film having a melt index of 3 and a density of 0.92 g/cm³. The ends of the backsheet were folded over the enveloped pad and attached with glue. Finally, the absorbent pad was covered with a topsheet of a hydrophobic but water and urine pervious material. (Webline No. F 6211 from the Kendall Co. of Walpole, Massachusetts, comprised of a non-woven rayon bonded with an acrylic latex).

Control diapers of the same design were made, using wood pulp fiber webs of 0.1 g/cm³ density (basis weight 960 g/m², core weight 36 g) instead of the absorbent structures of 0.3 g/cm³ density.

To test the performance of the diapers, the diapers were worn by normal infants in a leakage study.

The infants were allowed to play in a nursery school setting during the test. The diapers were left on the infants until leakage occurred. In order to speed up the test, the diapers were pre-loaded with a predetermined amount of synthetic urine.

After leakage occurred, the diapers were taken off and weighed to determine the amount of absorbed fluid. The loading X, defined as the amount of fluid (in grams) absorbed at the point that failure occurred per gram of absorbent material, was calculated.

The absorption capacity of the diapers containing the absorbent structure according to the present invention was equal to the capacity of the control diapers even though the core weight was 6 g less (30 g as compared to 36 g). Hence, with the absorbent structures of the present invention, a significant reduction in the consumption of wood pulp fibers can be obtained, without sacrificing absorption performance.

Similar diapers are prepared, using the sugar beet absorbent material of Example Two instead of the citrus peel absorbent material. Essentially the same results are obtained.

Example Four

Sanitary napkins employing an absorbent structure pursuant to this invention are prepared as follows:

An absorbent structure, prepared as in Example Three, is calendered to a density of 0.4 g/cm³ as measured under a confining pressure of 0.1 PSI ( 7 x 10³ N/m²). The web is cut into a pad of 8 in. x 2 in. ( . 20 cm x 5 cm) with tapered ends. On top of this pad is placed a second pad (rectangular) of 5 in. X 2 in. ( 13 cm x 5 cm). The combined pad structure is placed against a waterproof backing sheet (8 in. x 2 in., tapered) of embossed hard polyethylene having an embossed caliper of 2.3 mils. The structure is covered with a top sheet of non-woven, 3 denier needle punched polyester fabric having a density of 0.03 g/cm³ and a caliper of 2.3 mm. The thus covered structure is placed on a 9 in. x 3 in. ( 23 cm x 7.5 cm) bottom sheet of hydrophobic, spinbonded non-woven polyester having a measured weight of 15 g/m². The bottom sheet is prefolded upwardly by means of heat and pressure which bonds the superposed sheets together. The resulting absorbent structure is useful as a sanitary napkin and has excellent properties of absorption and containment of menses exudate.

### Example Five

A polyester-reinforced absorbent structure was prepared as follows.

2 g of 6 denier polyester fiber, fiber length 4 in. (about 10 cm) were carded and formed into an unbonded web. The web had a basis weight of 0.5 oz/yard² ( 19 g/m²). A web of 6 in. x 10 in. ( 15 cm x 25 cm) was placed on a wire screen which was covered with a tissue.

A mixture was formed of particles of a citrus peel absorbent material, (prepared as described in Example One) and particles of an acrylic acid grafted starch ("Sanwet 1M 1000", from Sanyo Co., Ltd., Japan) in a weight ratio 4:1; 15 g of this mixture were poured over the polyester fiber web, and forced into the web by reducing the air pressure under the wire screen. The resulting structure was compressed in a flat hydraulic press to a density of 0.3 g/cm³. The final composition was:

| | | |
|---|---|---|
| citrus peel absorbent material | 12 g | 76.4% |
| acrylic acid grafted starch | 3 g | 19.1% |

polyester fiber                                          0.7 g     4.5%

The absorbent structures were used for the preparation of diapers as described in Example Three.

The diapers containing these absorbent structures had a 15%-22% higher absorption capacity at the point of leakage than control diapers containing a wood pulp fiber web having a density of 0.1 g/cm³, and a basis weight of 960 g/m² (core weight of 36 g).

Hence, this embodiment of the present invention makes it possible to manufacture absorbent products which have a higher absorbent capacity and reduced bulk as compared to conventional diapers which contain a wood pulp fiber web as an absorbent core.

Absorbent structures are prepared using a 4:1 mixture of sugar beet absorbent material, prepared as described in Example Two, and a copolymer of isobutylene and maleic anhydride (KI Gel 201, from Kuraray, Japan); substantially similar results are obtained.

### Example Six

Sugar beet absorbent material prepared as described in Example Two was formed into diaper core test pads as follows:  A 4" x 4" (      10 x 10 cm) square of tissue was laid onto a flat surface.  On top of the tissue 2.42 grams of SAM was evenly spread.  Another 4" x 4" (      10 x 10 cm) square of tissue was laid on top.  Two such pads were prepared.  One was left uncompressed while the second was placed in a hydraulic press between flat steel plates and compressed to a density of 0.30 g/cm³.  The absorbency of the compressed pad was essentially equal to that of the uncompressed pad.

### Example Seven

Sugar beet absorbent material was prepared as described in Example Two, except that the drying step involved a solvent replacement with isopropanol.  The sugar beet absorbent material was mixed with southern slash pine Kraft pulp fibers and particles of a polyacrylate grafted starch water-insoluble hydrogel

- 30 -

(Sanwet IM 1000 from Sanyo Ltd., Japan. Approximate composition: starch: 10%; acrylic acid: 22%; sodium acrylate: 68%; methylene bisacrylamide (crosslinking agent): 0.2%).

The materials were mixed in the following quantities (by weight): wood pulp fibers: 50%; sugar beet absorbent material: 32%; polyacrylate grafted starch: 18%. The mixture was formed into a web having a basis weight of 0.144 g/m² by air-laying. The web was densified in a hydraulic press to a density of 0.25 g/cm³. The web was then cut into rectangular cores of 10 x 16 in. ( 25 x 40 cm). The cores were envelope-wrapped in wet strength tissue paper and incorporated into a diaper as described in Example Three.

0137608

<u>CLAIMS</u>

1. An absorbent structure comprising

(1)from 1% to 99% of a vegetable-derived absorbent material which comprises, by weight of the vegetable-derived absorbent material.

(a) from 15% to 60% pectin, less than 50% of which is in the form of a divalent metal salt;

(b) from 15% to 80% of a material selected from cellulose, hemicellulose, lignin, and mixtures thereof;

(c) from 0% to 4% chloroform soluble lipids; and

(d) from 0% to 35% non-lipid organic material extractable in a mixture of chloroform, methanol and water, said mixture having a volume ratio chloroform:methanol:water of 20:4:1; and

(2) from 1% to 99% of a conventional absorbent material.

2. An absorbent structure according to claim 1 wherein the pectin has a degree of esterification of from 1% to 45%.

3. An absorbent structure according to either one of claims 1 and 2 wherein less than 30% of the pectin is in the form of a calcium salt.

4. An absorbent structure according to any one of claims 1-3 wherein the vegetable-derived absorbent material comprises no more than 1% chloroform soluble lipids.

5. An absorbent structure according to any one of claims 1-4 wherein the vegetable-derived absorbent material comprises no more than 10% of component (d).

6. An absorbent structure according to any one of claims 1-5 wherein the vegetable-derived absorbent material comprises from 0% to 6% water-soluble metal salts.

7. An absorbent structure according to any one of claims 1-6 wherein the vegetable-derived absorbent material is prepared from citrus peels, sugar beet pulp, apple pulp, or mixtures thereof.

8. An absorbent structure according to claim 7 wherein component (1) is a citrus peel-derived absorbent material which comprises, by weight of the citrus peel-derived absorbent material

    (a) from 30% to 60% pectin, said pectin having a degree of esterification of less than 20%, and less than 30% of the pectin being in the form of a calcium salt;

    (b) from 30% to 60% of a mixture of cellulose and hemicellulose;

    (c) from 0% to 1% chloroform soluble lipids;

    (d) from 0% to 10% non-lipid organic materials extractable in said mixture of chloroform, methanol and water, and

    (e) from 0% to 6% water-soluble metal salts;

9. An absorbent structure according to claim 7 wherein component (1) is a sugar beet-derived absorbent material which comprises, by weight of the sugar beet-derived material:

    (a) from 15% to 35% pectin, said pectin having a degree of esterification of less than 45%, and less than 30% of the pectin being in the form of a calcium salt;

    (b) from 20% to 80% of a mixture of cellulose and hemicellulose;

    (c) from 0% to 1% chloroform soluble lipids;

    (d) from 0% to 10% non-lipid organic materials extractable in said mixture of chloroform, methanol and water, and

    (e) from 0% to 6% water-soluble metal salts;

1979 to Mitchell et al. Another attempt at converting citrus waste to a food additive for human consumption is disclosed in U.S. Patent 4,225,628, issued September 30, 1980 to Lynn. According to the process described in this reference, citrus peel particles are de-watered by a process very similar to the lime de-watering process used in cattle feed production; the material is subsequently mixed with sesame grain flour, ground, dried and milled to a desired particle size. U.S. Patent 4,379,782, issued April 12, 1983 to Staub et al. discloses the use of citrus albedo or sugar beet pulp as a dietary fiber. The material is extracted with water or isopropanol to remove soluble carbohydrates and color and flavor materials. In spite of these attempts at finding more profitable uses for citrus waste, almost all of the citrus waste from juice canneries is still being converted to cattle feed and sold at a price which barely provides for recovery of the processing costs.

Sugar beet residue (commonly referred to as beet pulp), like citrus residue, is generally converted to cattle feed. As for citrus residues, attempts have been reported to convert beet pulp into a food additive suitable for human consumption. An example is Japanese Patent SHO 57-54573, publication date April 1, 1982. This patent discloses a method for upgrading beet pulp by bleaching the pulp in hypochloric acid at pH 6.5 to 7.5, washing with water and drying. The material is reported to be capable of absorbing about 90% of its weight in water.

## Summary of the Invention

The present invention relates to absorbent structures comprising (1) from 1% to 99% of a vegetable-derived absorbent material which comprises by weight of the vegetable-derived absorbent material (a) from 15% to 60% pectin, less than 50% of which is in the form of a divalent metal salt; (b) from 15% to 80% of a material selected from cellulose, hemicellulose, lignin, and mixtures thereof; (c) from 0% to 4% chloroform soluble lipids; (d) from 0% to 35% non-lipid organic materials extractable in a mixture of chloroform, methanol and water, said mixture having a volume ratio chloroform:methanol:water of 20:4:1; and (2) from 1% to 99% of a conventional absorbent material.

The invention further relates to disposable absorbent products, such as diapers or napkins, comprising the absorbent structures of the present invention. Such absorbent products typically comprise (a) a liquid impervious backing sheet; (b) a hydrophobic top sheet; and (c) an absorbent structure according to the present invention, said structure being placed between the backing sheet and the top sheet.

## Brief Description of the Drawing

Fig. 1 represents a flow chart of the analytical protocol used in characterizing the vegetable-derived absorbent materials used in the absorbent structures.

## Detailed Description of the Invention

This invention relates to novel absorbent structures comprising a novel vegetable absorbent material, and to absorbent products comprising said absorbent structures.

This invention is based upon the discovery that pectin-containing vegetable materials can be converted into absorbent materials, using a relatively simple and inexpensive process. Pectin-containing vegetable materials which are suitable as starting materials for the production of the absorbent materials of the present invention contain at least 15% pectin. Examples include apples, apricots, citrus peels, sugar beets and watermelon rinds. Zucchini for example, which has a pectin content of 8%, is not suitable. Citrus peels and sugar beet pulp, each of which is a by-product of an important agricultural industry, are available in large quantities and at low cost and are therefore preferred starting materials for the preparation of the absorbent materials of the present invention.

The composition parameters of the vegetable starting materials and of the vegetable absorbent materials obtained upon processing have been determined in the comprehensive analytical protocol given below. Percentages of components of the materials, as used herein, are weight percentages as determined

1/1

0137608